# EUROPÄISCHE PATENTANMELDUNG

(11) **EP 1 779 792 A1**
(43) Veröffentlichungstag der Anmeldung: **02.05.2007**
(21) Anmeldenummer: 06017497.6
(22) Anmeldetag: 23.08.2006
(51) Int. Cl.: A61B 17/16, A61B 17/00

(54) **Facettengelenkfräser**

(30) Priorität: 26.10.2005 DE 202005016761 U
(71) Anmelder: Joimax GmbH, 76227 Karlsruhe (DE)
(72) Erfinder: Ries, Wolfgang, 76351 Linkenheim (DE); Notheis, Mathias, 76694 Forst (DE)
(74) Vertreter: Lempert, Jost

(57) **Zusammenfassung**

Bei einem Facettengelenkfräser mit einem im wesentlichen zylindrischen Schaft, mit einer am distalen Ende des Schafts vorgesehenen Zähnung und mit einem Griff am proximalen Ende, sind Schaft und Griff trennbar.

## Beschreibung

Die Erfindung betrifft einen Facettengelenkfräser mit einem im Wesentlichen zylindrischen Schaft, mit einer am distalen Ende des Schafts vorgesehenen Zähnung und mit einem Griff am proximalen Ende.

Ein gattungsgemäßer Facettengelenkfräser ist aus der DE 699 17 683 T2 bekannt. Der bekannte Fräser weist einen hohlen zylindrischen Schaft, an seinem rückwärtigen, proximalen Ende einen Handgriff und an seinem stirnseitigen Ende eine Zähnung auf.

Ein derartiger Fräser wird zum Ausfräsen von Wirbelbestandteilen im Bereich eines seitlichen Fortsatzes eines Wirbelsäulen-Wirbels eingesetzt, um einen postero-lateralen Zugang zu eingeklemmten Nervenwurzeln des zentralen Nervensystems zu schaffen. Durch diesen Zugang werden dann Nucleus-Pulposus-Gewebe und andere Gewebearten (Kapselgewebe, Narbengewebe, Annulus-Gewebe) entfernt, da diese auf die Nervenwurzeln drücken. Der fragliche Fortsatz eines Wirbels bildet mit einem benachbarten Fortsatz eines benachbarten Wirbels das sogenannte Facettengelenk.

Die einen gattungsgemäßen Facettengelenkfräser einsetzende mikroinvasive Operationstechnik zur Dekompression von eingeklemmten Nervenwurzeln ist höchst erfolgreich. Allerdings hat sich herausgestellt, dass im Bereich der Zähnung auch bei üblicher Reinigung und Desinfektion Knochengewebe verbleiben kann, das bestenfalls mit einem erheblichen Aufwand sorgfältig entfernt werden könnte. Darüber hinaus ist, da Fräser unterschiedlicher Stärken eingesetzt werden, wünschenswert, unterschiedliche Fräser mit einem gemeinsamen Betätigungsgriff einsetzen zu können.

Der Erfindung liegt daher die Aufgabe zugrunde, einen gattungsgemäßen Facettengelenkfräser in der vorstehend angegebenen Weise zu verbessern.

Zur Lösung der genannten Aufgabe sieht die Erfindung einen gattungsgemäßen Facettengelenkfräser vor, der dadurch gekennzeichnet ist, dass Schaft und Griff trennbar sind, wobei insbesondere Schaft und Griff zerstörungsfrei trennbar sind.

In konkreter Ausgestaltung kann hierzu vorgesehen sein, dass Schaft und Griff mit einer zusammenwirkenden Verbindungseinrichtung versehen sind, wobei die Verbindungseinrichtung derart ausgebildet ist, dass der Schaft an seinem proximalen Ende eine seitliche Vertiefung aufweist, in die eine am Griff gelagerte Kugel eingreift, die wiederum in einer Ausnehmung am Fräserschaft einbringbar ist. Die Kugel kann am Griff blockiert werden, so dass damit der Fräserschaft im Griff sitzungssicher gehalten ist. Dies geschieht dadurch, dass ein Schieber zwischen einer Freigabestellung zur Freigabe des Schafts vom Griff und einer Blockierstellung, in der der Schaft im Griff zuverlässig gehalten ist, bewegbar ist.

In einer bevorzugten Weiterbildung ist dabei vorgesehen, dass der Schieber federbelastet ist, wobei insbesondere der Schieber durch eine Feder in der Blockierstellung gehalten wird.

Eine Weiterbildung sieht vor, dass der Schieber einen erweiterten inneren Bereich aufweist. Hierdurch wird ein Bereich im Schieber gebildet, in den die Kugel, wenn dieser über die Kugel gelangt, zurücktreten kann, so sich hier-durch eine Freigabestellung für den Fräserschaft ergibt und dieser daher bei der genannten Position des Schiebers und der Kugel aus dem Handgriff entfernt werden kann.

Zusätzlich ist in bevorzugter Ausgestaltung weiterhin vorgesehen, dass der Schieber aus seiner Blockierstellung entgegen der Kraft der Feder durch Einwirken von Hand in seine Freigabestellung verschiebbar ist, wobei entsprechend den unterschiedlichen Vorlieben von Operateuren vorgesehen sein kann, dass entweder der Schieber entgegen der Wirkung der Feder in proximaler Richtung in seine Freigabestellung verschiebbar ist oder aber der Schieber entgegen der Wirkung der Feder in distale Richtung in seine Freigabestellung verschiebbar ist.

Weitere bevorzugte Ausgestaltungen der Erfindung zeichnen sich dadurch aus, dass Schaft und Handgriff drehfest miteinander verbindbar sind, wobei insbesondere ein Verbindungsteil des Schaftes als Außen-Mehrkant, insbesondere Dreikant und ein Aufnahmebereich des Griffs als Innen-Mehrkant, vorzugsweise Innen-Dreikant, ausgebildet ist.

In äußerst bevorzugter Ausgestaltung ist vorgesehen, dass ein Verbindungsteil des Schaftes aus Kunststoff besteht, wobei das Verbindungsteil fest und unlösbar (zumindest nicht zerstörungsfrei lösbar) mit dem Fräserschaft verbunden, beispielsweise um diesen herum gespritzt ist. Hierdurch können verschiedene Schaftstärken kodiert werden, beispielsweise durch unterschiedliche Farben des Kunststoffteils, also Ausbildung desselben mit Reflektions-Absorptionsvermögen in unterschiedlichen Wellenlängenbereichen. Darüber hinaus kann an den Kunststoffschacht eine Markierung, beispielsweise in Form einer Erhöhung vorgesehen sein, wenn der Fräserschaft mit einer die Zähnung überragenden seitlichen Schutzlippe zum Schutz von Nerven beim Arbeiten vorgesehen ist, wobei diese Markierung von einer Erhöhung auf dem Kunststoffteil mit der Lippe am distalen Ende des Fräsers fluchtet.

Weitere Vorteile und Merkmale der Erfindung ergeben sich aus den Ansprüchen und aus der nachfolgenden Beschreibung, in der zwei bevorzugte Ausführungsformen des erfindungsgemäßen Facettengelenkfräsers dargestellt sind. Dabei zeigt:
- Fig. 1: eine erste Ausgestaltung des erfindungsgemäßen Facettengelenkfräsers, teilweise im Längsschnitt;
- Fig. 2: eine andere Ausgestaltung eines erfindungsgemäßen Facettengelenkfräsers, ebenfalls teilweise im Längsschnitt; und

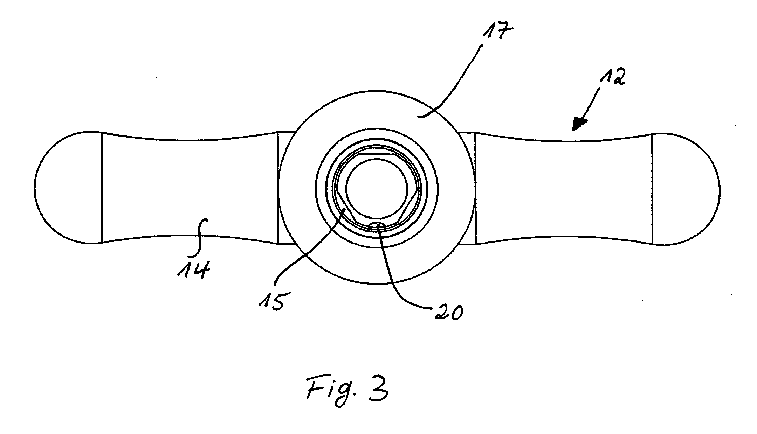

- Fig. 3: eine Stirnansicht auf die Einführungsöffnung des Handgriffs eines erfindungsgemäßen Facettengelenkfräsers.
- Fig. 4: eine Rückansicht zweier benachbarter Lendenwirbel der menschlichen Wirbelsäule; und
- Fig. 5: eine teilweise geschnitten dargestellte Ansicht der spinalen Bandscheiben zwischen den beiden Wirbeln gemäß Fig. 4.

Der erfindungsgemäße Facettengelenkfräser 1 weist einen im Wesentlichen zylindrischen Schaft 2 aus Stahl auf, der an seinem distalen Ende 3 mit einer Zähnung 4 versehen ist. Am proximalen Ende 5 weist der Schaft ein Befestigungsteil 6 in Form einer auf ihn aufgespritzten Mehrkant-, hier Dreikant-Kunststoffhülse auf, die in ihrer Seitenwandung mit einer Vertiefung 7 versehen ist.

Der Schaft 2 ist mit einem Griff 11 zerstörungsfrei lösbar verbindbar.

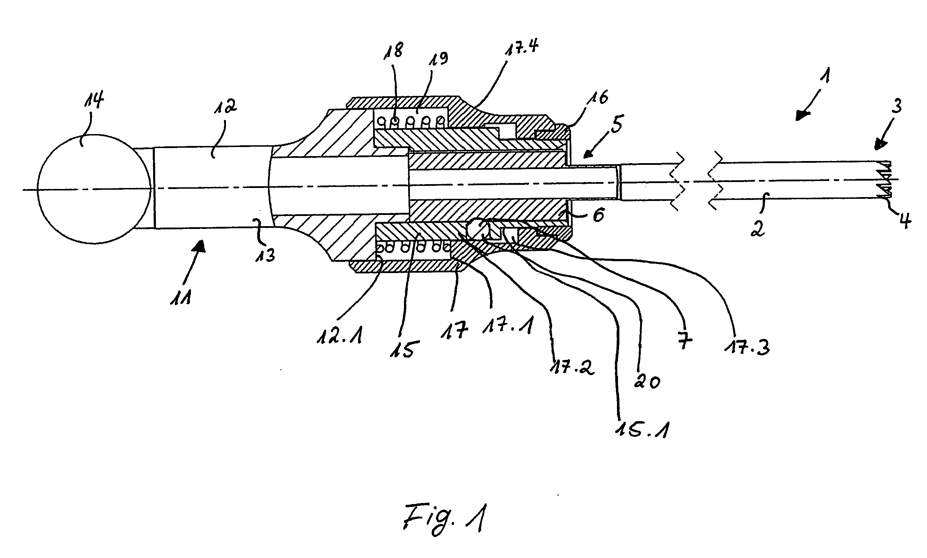

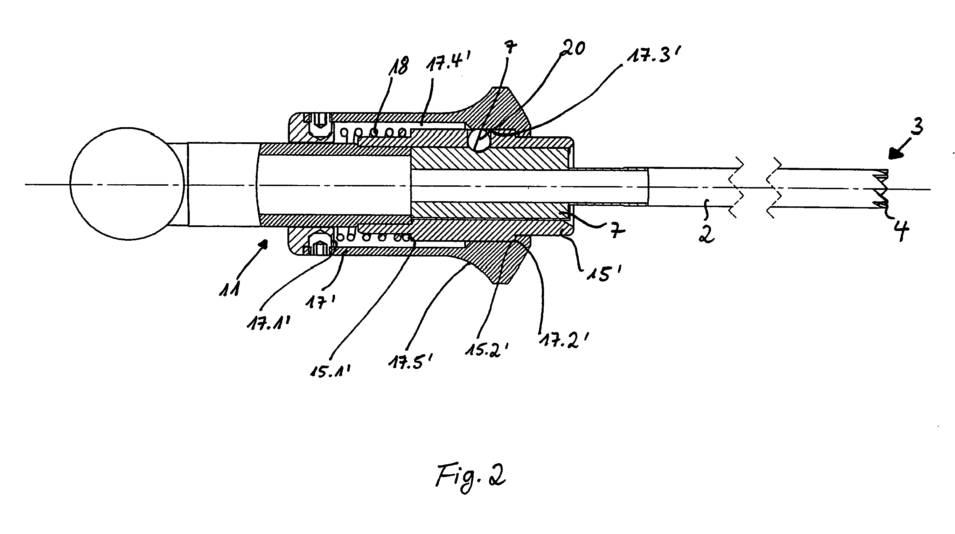

Der Griff 11 weist ein T-förmiges Griffteil 12 mit einem Längssteg 13 und an dessen distalen Ende einen Quersteg 14 auf. Mit dem Griffteil 12, genauer dem proximalen Ende des Längsstegs 13 ist fest eine Hülse 15 verbunden, an deren Ende wiederum ein Abschlussstück 16 aufsitzt. Entlang der Hülse ist ein Schieber 17 axial entgegen der Wirkung einer Feder 18 verschiebbar, die in einer Ausnehmung 19 zwischen Hülse 15 und Schieber 17 angeordnet ist und ihr Widerlager im dargestellten Ausführungsbeispiel der Fig. 1 einerseits - proximal - an einem stirnseitigen Ringabschnitt 12.1 des Griffteils 12 und andererseits an einer ringartigen Schulter 17.1 des Schiebers 17 findet, so dass die Feder diesen in distale Richtung vorbelastet bzw. drückt.

In einer Ausnehmung der Hülse 15 befindet sich eine Kugel 20, die radial innenseitig durch einen die Ausnehmung begrenzenden Ringflansch 15.1 gehalten wird, so dass sie aus der Hülse 15 nicht herausfallen kann. Der Schieber 17 weist einen auf der Hülse 15 gleitenden Bereich 17.2 auf, mittels dessen die Hülse, wie dies in der Fig. 1 dargestellt ist, in die Ausnehmung 7 des Teils 6 hineingedrückt wird. Dem schließt sich auf der der Feder abgewandten Seite eine Ausnehmung 17.3 an, die größere radiale Abmessungen hat als die Wandung 17.2 und damit, wenn der Bereich 17.3 auf die axiale Höhe der Kugel gelangt, diese freigibt, so dass der Schaft 2 aus dem Griff 11 herausgezogen werden kann.

Auf der im Querschnitt 14 des Griffs 11 abgewandten Seite des Schiebers 17 befindet sich eine Griffmulde 17.4. Der Schieber 4 kann entgegen der Wirkung der Feder 18 durch Angreifen an der Griffmulde 17.4 und Ziehen des Schiebers entgegen dem Querschnitt 14 bewegt werden, woraufhin die erweiterte Ausnehmung 17.3 des Schiebers auf die axiale Höhe der Kugel 20 gelangt und derart diese zum Herausnehmen oder Einsetzen des Fräsers 2 freigibt. Wird der Schieber 17 wieder freigegeben, so schiebt sein Bereich 17.2 unter Einfluss der Feder 18 sich über die Kugel 20, drückt diese damit in die Ausnehmung 7 und blockiert derart den Fräser 2 in der hier durchgegebenen axialen Stellung.

Durch die Mehrkantausbildung von Verbindungsteil 6 und Ausnehmung des Griffs 11 ist der Fräser ebenfalls drehfest im Griff 11 festgelegt.

Die Fig. 2 zeigt eine andere Ausgestaltung des erfindungsgemäßen Fräsers 1. Gleiche Teile sind mit gleichen Bezugszeichen versehen. Zur Beschreibung wird grundsätzlich auf die vorstehenden Ausführungen verwiesen. Lediglich Unterschiede werden im Folgenden im Detail erläutert.

Auch bei der Ausgestaltung der Fig. 2 ist am Griff 11 eine Hülse 15' vorgesehen und von einem entlang derselben in axialer Richtung verschiebbaren Schieber 17' umgeben. Am rückwärtigen, proximalen Ende der Hülse 15' ist eine Schulter 15.1' ausgebildet zwischen derer eine ebenfalls proximal am Schieber 17' ausgebildeten nach innen ragenden Ringabsatz 17.1' wiederum eine Schraubenfeder 18 angeordnet ist. Diese belastet also den Schieber 17' nach rückwärts oder in proximaler Richtung. Der Schieber findet einen Anschlag über kooperierende Schultern 15.2' an der Hülse 15' und 17.2' am Schieber.

In einer Ausnehmung der Hülse 15' ist wiederum eine Kugel 20 in der beschriebenen weise gelagert. Der Schieber 17' weist einen Bereich 17.3' auf, dessen Innendurchmesser mit dem Außendurchmesser der Hülse 15 in diesem Bereich fluchtet. Proximal zu dem Bereich 17.3' ist ein erweiterter Bereich 17.4' des Schiebers vorgesehen.

Durch die Feder 18 wird der Schieber in die in der Fig. 2 dargestellte Position gedrückt, bei der der Bereich 17.3' des Schiebers 17' sich auf axialer Höhe der Kugel 20 findet und diese in die Ausnehmung 7 des Verbindungsteils 6 hineindrückt und den Schaft 2 so im Griff 11 blockiert.

Am Schieber ist weiterhin ein Griffteil 17.5' ausgebildet, mit dem der Schieber in distale Richtung verschoben werden kann.

Greift der Operateur am Griffbereich 17.5 an und schiebt den Schieber 17' in distale Richtung, so gelangt der erweiterte Bereich 17.4' des Schiebers 17' auf axiale Höhe der Kugel 20, so dass diese sich radial nach außen bewegen kann, aus der Ausnehmung 7 austritt und damit der Schaft 2 aus dem Griff 7 entfernt werden kann bzw. ebenfalls bei dieser Position des Schiebers ein Schaft 2 in den Griff 11 eingeführt werden kann.

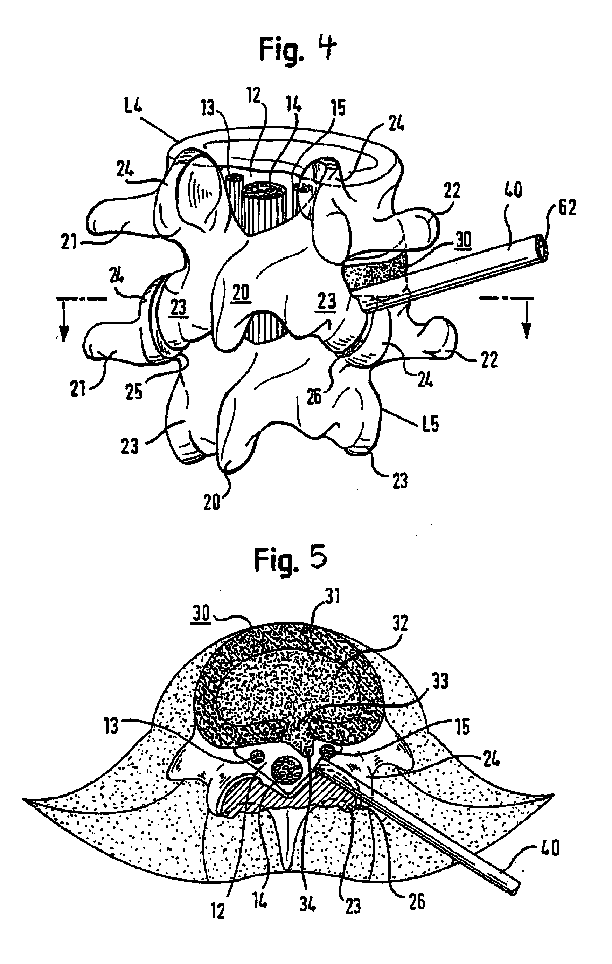

Der erfindungsgemäße Fräser wird in der im Folgenden unter Bezug auf die Fig. 4 und 5 beschriebenen Weise eingesetzt.

Die Fig. 4 zeigt beispielhaft den vierten und fünften Lendenwirbel L4, L5 und zwischen diesen eine Bandscheibe 30 mit einer Einklemmung 33 am Faserknorpelring 31 (Annulus fibrosus) unmittelbar zur Rechten der Mittelachse der Wirbelsäule mit einer Extrusion 34 von Nucleus pulposus-Gewebe in den Canalis vertebralis - Fig. 5.

Im Inneren des Wirbelkanals 22 (Canalis vertebralis) sind schematisch Nervenstrukturen 23, 24, 25 dargestellt. Jeder Wirbel L4, L5 weist einen Dornfortsatz 40 und einen linken und rechten Querfortsatz 42, linke und rechte untere, ein Gelenk bildende Fortsätze 43 sowie linke und rechte obere, ein Gelenk bildende Fortsätze 44 auf, wobei das linke und das rechte Gelenk zwischen dem oberen und dem unteren Lendenwirbel L4, L5, das als Facettengelenk 46 bezeichnet wird, durch jeweils die unteren Fortsätze 43 des oberen Wirbels L4 und die oberen Fortsätze 44 des unteren Wirbel L5 gebildet ist.

Das Arbeiten mit dem erfindungsgemäßen Fräser gestaltet sich in der folgenden Weise:

Zunächst wird eine Hohlnadel mit einem Außendurchmesser in der Größenordnung von 1,25 mm in eine der Einklemmung benachbarten Position vorgeschoben. Sodann wird ein Leitdraht durch das Lumen der Hohlnadel geschoben, bis sein distales Ende das Ende der Hohlnadel etwas überragt. Anschließend wird die Hohlnadel entnommen, wogegen der Leitdraht am Ort verbleibt. Es wird sodann eine Trokar-Röhre über den Leitdraht vorgeschoben, bis das stumpfe Ende des Trokars sich an dem Facettengelenk 46 befindet. Unter Halten des Trokars in dieser Position wird ein erfindungsgemäßer Fräser mit einem geringen Durchmesser in der Größenordnung von etwa 4,5 mm über den Trokar 54 vorgeschoben, bis das distale Ende des Fräsers an der Oberfläche des Facettengelenks 46 zur Anlage kommt. Nun dreht der Operateur an dem ihm nahen (proximalen) Ende des Fräsers den dort vorgesehenen Handgriff, beispielsweise von Hand, so dass im Vorsprung 43 des Wirbels L4 ein Kanal erzeugt wird. Dieser Schritt wird mit Trokar-Röhren und Fräsern größeren Durchmessers wiederholt, bis ein hinreichender Durchmesser des Kanals erreicht ist, um eine Kanüle mit einem Lumen aufzunehmen, das hinreichend groß ist, um nicht nur ein Zangenwerkzeug, sondern auch ein Endoskop hindurchführen zu können. Anschließend wird mit dem Zangenwerkzeug, gegebenenfalls unter Endoskopbeobachtung, die Einklemmung entfernt.

### Bezugszeichenliste

- 1: Facettengelenkfräser
- 2: Schaft
- 3: distales Ende
- 4: Zähnung
- 5: proximales Ende
- 6: Befestigungsteil
- 7: Vertiefung

- 11: Griff
- 12: T-förmiges Griffteil
- 12.1: Ringabschnitt
- 13: Längssteg
- 14: Quersteg
- 15: Hülse
- 15.1: Ringflansch
- 15': Hülse
- 15.1': Schulter
- 15.2': Schulter
- 16: Abschlussstück
- 17: Schieber
- 17.1: ringartige Schulter
- 17.2: Wandung
- 17.3: Ausnehmung
- 17.4: Griffmulde
- 17.5: Griffbereich
- 17': Schieber
- 17.1': Ringabsatz
- 17.2': Schulter
- 17.3': Bereich
- 17.4': erweiterter Bereich
- 17.5': Griffteil
- 18: Schraubenfeder
- 19: Ausnehmung
- 20: Kugel

- 22: Wirbelkanal
- 23, 24, 25: Nervenstrukturen

- 30: Bandscheibe
- 31: Faserknorpelring
- 33: Einklemmung
- 34: Extrusion

- 40: Dornfortsatz
- 42: Querfortsatz
- 43: Fortsätze
- 44: Fortsätze
- 46: Facettengelenk

- 54: Trokar

- L4, L5: Lendenwirbel

## Patentansprüche

1. Facettengelenkfräser mit einem im Wesentlichen zylindrischen Schaft, mit einer am distalen Ende des Schafts vorgesehenen Zähnung und mit einem Griff am proximalen Ende, **dadurch gekennzeichnet, dass** Schaft (2) und Griff (11) trennbar sind.

2. Fräser nach Anspruch 1, **dadurch gekennzeichnet, dass** Schaft (2) und Griff (11) zerstörungsfrei trennbar sind.

3. Fräser nach Anspruch 1 oder 2, **dadurch gekennzeichnet, dass** Schaft (2) und Griff (11) mit einer zusammenwirkenden Verbindungseinrichtung versehen sind.

4. Fräser nach einem der vorangehenden Ansprüche, **dadurch gekennzeichnet, dass** der Schaft (2) an seinem proximalen Ende (5) eine seitliche Vertiefung (6) aufweist, in die eine am Griff (11) gelagerte Kugel (20) eingreift.

5. Fräser nach einem der vorangehenden Ansprüche, **dadurch gekennzeichnet, dass** ein Schieber (17, 17') zwischen einer Freigabestellung zur Freigabe des Schafts (2) vom Griff (11) und einer Blockierstellung, in der der Schaft (2) im Griff (11) zuverlässig gehalten ist, bewegbar ist.

6. Fräser nach Anspruch 5, **dadurch gekennzeichnet, dass** der Schieber federbelastet ist.

7. Fräser nach Anspruch 6, **dadurch gekennzeichnet, dass** der Schieber durch eine Feder (18) in der Blockierstellung gehalten wird.

8. Fräser nach einem der vorangehenden Ansprüche, **dadurch gekennzeichnet, dass** der Schieber einen erweiterten inneren Bereich aufweist.

9. Fräser nach einem der vorangehenden Ansprüche, **dadurch gekennzeichnet, dass** der Schieber aus seiner Blockierstellung entgegen der Kraft der Feder (18) durch Einwirken von Hand in seine Freigabestellung verschiebbar ist.

10. Fräser nach einem der vorangehenden Ansprüche, **dadurch gekennzeichnet, dass** der Schieber entgegen der Wirkung der Feder (18) in proximaler Richtung in seine Freigabestellung verschiebbar ist.

11. Fräser nach einem der Ansprüche 1 bis 9, **dadurch gekennzeichnet, dass** der Schieber entgegen der Wirkung der Feder (18') in distale Richtung in seine Freigabestellung verschiebbar ist.

12. Fräser nach einem der vorangehenden Ansprüche, **dadurch gekennzeichnet, dass** Schaft (2) und Handgriff (11) drehfest miteinander verbindbar sind.

13. Fräser nach Anspruch 12, **dadurch gekennzeichnet, dass** ein Verbindungsteil (6) des Schaftes (2) als Außen-Mehrkant, insbesondere Dreikant und ein Aufnahmebereich des Griffs (11) als Innen-Mehrkant, vorzugsweise Innen-Dreikant, ausgebildet ist.

14. Fräser nach einem der vorangehenden Ansprüche, **dadurch gekennzeichnet, dass** ein Verbindungsteil des Schaftes aus Kunststoff besteht.
